**Europäisches Patentamt**

**European Patent Office**

**Office européen des brevets**

(11) Veröffentlichungsnummer: **0 022 579 B1**

# EUROPÄISCHE PATENTSCHRIFT

(45) Veröffentlichungstag der Patentschrift:
**29.10.86**

(21) Anmeldenummer: **80104079.1**

(22) Anmeldetag: **14.07.80**

(51) Int. Cl.⁴: **A 61 B 6/02,** A 61 B 6/04, A 61 B 6/06

(54) Schichtaufnahmegerät zur Herstellung von Transversalschichtbildern.

(30) Priorität: **17.07.79 DE 2928825**

(43) Veröffentlichungstag der Anmeldung:
**21.01.81 Patentblatt 81/3**

(45) Bekanntmachung des Hinweises auf die Patenterteilung:
**29.10.86 Patentblatt 86/44**

(84) Benannte Vertragsstaaten:
**FR GB**

(56) Entgegenhaltungen:
**DE - A - 2 613 809**
**DE - A - 2 655 661**
**DE - A - 2 732 073**
**FR - A - 2 299 845**
**FR - A - 2 330 370**
**FR - A - 2 344 032**
**FR - A - 2 408 197**
**US - A - 3 974 388**
**US - A - 4 034 224**
**US - A - 4 097 746**
**US - A - 4 131 802**

(73) Patentinhaber: **Siemens Aktiengesellschaft Berlin und München, Wittelsbacherplatz 2, D-8000 München 2 (DE)**

(72) Erfinder: **Kalender, Willi, Dr., Neue Strasse 4, D-8521 Möhrendor (DE)**
Erfinder: **Linke, Gerhard, Dipl.-Phys., Weiherstrasse 17, D-8520 Erlangen (DE)**
Erfinder: **Pfeiler, Manfred, Dr. Dipl.-Ing., Ludwig-Thoma-Strasse 25, D-8520 Erlangen (DE)**

ACTORUM AG

**Beschreibung**

Die Erfindung betrifft ein Schichtaufnahmegerät gemäss dem Oberbegriff des Patentanspruches.

Zur Herstellung von Transversalschichtbildern sind Schichtaufnahmegeräte dieser Art, sogenannte Computer-Tomographen, bekannt, die eine Strahlenmessanordnung aufweisen, mit der die Transversalschicht unter verschiedenen Projektionen abgetastet wird. Aus den Ausgangssignalen des Strahlenempfängers der Strahlenmessanordnung berechnet ein Computer das Bild der untersuchten Transversalschicht. Zur definierten Wahl der untersuchten Transversalschicht ist es bei einem solchen Computer-Tomographen notwendig, die Patientenliege gegen Längsverschiebung zu verriegeln (DE-A-2 655 661). Bei verriegelter Patientenliege wird dabei aber dann der Patient durch Drehung der Strahlenmessanordnung abgetastet. Es ist auch bei einem Computer-Tomographen bekannt, die Breite des Strahlenbündels in der Schichtebene durch eine verstellbare Blende einzugrenzen (DE-A-2 609 925).

Bei bestimmten röntgendiagnostischen Untersuchungen unter Anwendung von Kontrastmitteln benötigt der Arzt quantitative Angaben über die Kontrastmittelkonzentration und ihren zeitlichen Verlauf, z.B. in grösseren Blutgefässen.

Der Erfindung liegt die Aufgabe zugrunde, einen Computer-Tomographen so auszubilden, dass mit ihm eine Untersuchung der Kontrastmittelkonzentration und ihres zeitlichen Verlaufes in einem Gefäss oder im Organgewebe möglich ist.

Bei der Lösung dieser Aufgabe ist von einem Röntgenschichtgerät ausgegangen, wie es in der DE-A-2 613 809 beschrieben ist. Bei diesem bekannten Computertomographen ist es möglich, die Messanordnung gegen Drehung während der Verarbeitung der Ausgangssignale des Strahlenempfängers zu verriegeln. Diese Verriegelung hat dabei jedoch den Zweck, mit Hilfe einer Längsverschiebung der Patientenliege ein Röntgenschattenbild zu erzeugen. Der Verlauf eines Kontrastmittels an einer bestimmten Stelle der untersuchten Schicht ist dabei nicht erfassbar.

Die Aufgabe ist erfindungsgemäss durch die im kennzeichnenden Teil des Patentanspruches angegebene Ausbildung gelöst. Bei dem erfindungsgemässen Schichtaufnahmegerät bleibt während der Verarbeitung der Messsignale sowohl die Messanordnung als auch die Patientenliege verriegelt. Es ist daher möglich, den zeitlichen Verlauf der Kontrastmittelkonzentration an einer bestimmten Stelle der durchstrahlten Schicht exakt zu verfolgen. Dabei ist das Röntgenstrahlbündel mittels einer verstellbaren Blende auf eine Breite eingeblendet, die dem gewünschten zu untersuchenden Schichtbereich entspricht. Die Strahlenbelastung des Patienten ist daher äusserst niedrig. Es können alle Detektorelemente für die Herstellung eines üblichen Computertomographiebildes herangezogen werden, während für die Verfolgung des Kontrastmittelverlaufes nur die jeweils erforderlichen Detektorelemente benutzt werden.

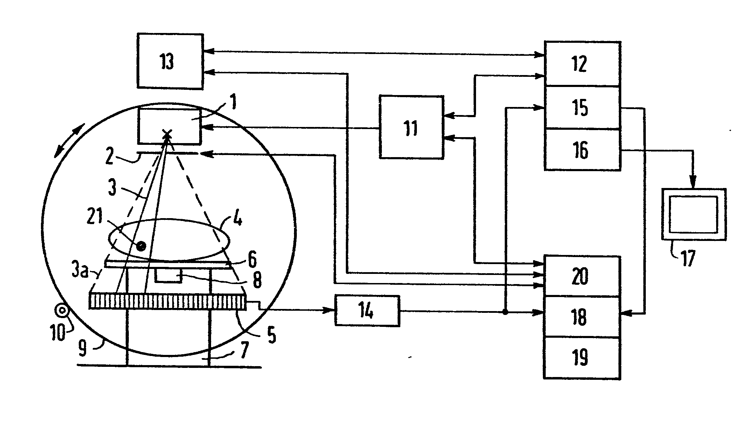

Die Erfindung ist nachfolgend anhand eines in der Zeichnung dargestellten Ausführungsbeispieles näher erläutert.

In der Zeichnung ist eine Röntgenröhre 1 dargestellt, die ein mittels einer Blende 2 eingeblendetes Röntgenstrahlenbündel 3 erzeugt, das einen Patienten 4 in einer Transversalschicht durchdringt. Die Abmessung des Röntgenstrahlenbündels 3 senkrecht zur Schichtebene ist gleich der Schichtstärke. Die aus dem Patienten 4 austretende Röntgenstrahlung trifft auf einem Strahlenempfänger 5 auf, der aus einer Reihe von Detektorelementen besteht und eine solche Abmessung hat, dass er bei vollständiger Bestrahlung, also bei vollständig geöffneter Blende 2, den abzubildenden Querschnitt des Patienten 4 erfasst. Das Röntgenstrahlenbündel ist für diesen Fall gestrichelt gezeichnet und mit 3a bezeichnet.

Der Patient 4 ruht auf einer Patientenliege 6, die mit einem Sockel 7 verbunden ist und durch eine Verriegelungsvorrichtung 8 gegen Längsverschiebung verriegelt werden kann. Die Messanordnung aus der Röntgenröhre 1 und dem Strahlenempfänger 5 ist auf einem schematisch dargestellten Drehring 9 angeordnet, der durch einen Antrieb 10 in Pfeilrichtung zur Abtastung des Patienten 4 unter verschiedenen Projektionen gedreht werden kann. Der Antrieb 10 kann den Drehring 9 und damit die Messanordnung 1, 5 in einer bestimmten Projektion verriegeln.

Die Röntgenröhre 1 wird von einem Röntgengenerator 11 gespeist, der an einer Steuereinrichtung 12 angeschlossen ist, die auch eine Geräteelektronik 13 steuert. Der Strahlenempfänger 5 ist an einer Messwerterfassungseinheit 14 angeschlossen, die einen Rechner 15 ansteuert, der aus den von der Messwerterfassungseinrichtung 14 gelieferten Daten ein Bild der untersuchten Transversalschicht des Patienten 4 berechnet. Das berechnete Bild kann in einem Bildspeicher 16 gespeichert und auf einem Sichtgerät 17 optisch wiedergegeben werden. Die Messdaten der Messwerterfassungseinrichtung 14 werden zur Bildrekonstruktion während der Drehung der Messanordnung 1, 5 um einen Winkel von 360° um den Patienten 4 unter verschiedenen Projektionen gewonnen.

Die Messwerterfassungseinheit 14 ist an einem weiteren Rechner 18 angeschlossen, der für die Bestimmung der Kontrastmittelkonzentration in einem bestimmten Bereich der untersuchten Transversalschicht dient. Die vom Rechner 18 bestimmten Werte können in einem Speicher 19 gespeichert werden. Er arbeitet mit einer Steuereinrichtung 20 zusammen.

Für die Bestimmung der Kontrastmittelkonzentration wird zunächst eine übliche Abtastung des Patienten 4 vorgenommen und mittels des Rechners 15 ein Bild der untersuchten Transversalschicht bestimmt. Dabei kann beispielsweise der Querschnitt eines Blutgefässes, in dem die Kontrastmittelkonzentration bestimmt werden soll, berechnet werden. Eine entsprechende Information über diesen Querschnitt gibt der Rechner 15 an den Rechner 18. Anschliessend wird mittels der Blende 2 das Röntgenstrahlenbündel, das ursprünglich zur Bilderzeugung den gesamten Strahlenempfänger 5 getroffen hat, von seiner Grösse 3a auf die Grösse 3 eingeblendet. Die Blendeneinstellung erfolgt dabei von der Steuereinrichtung 20, entsprechend dem vom Benutzer eingegebenen Bereich des Strahlenempfängers 5, der die Röntgenstrahlung messen soll. Es durchsetzt die Transversalschicht des Patienten 4 somit nur im Bereich eines Blutgefässes 21. Ferner wird mittels der Antriebsvorrichtung 10 der Drehring 9 gegen Drehung und die Patientenliege 6 mittels der Verriegelungsvorrichtung 8 gegen Längsverschiebung verriegelt. Bei ruhendem Patienten 4 und ruhender Messanordnung 1, 5 werden die Messwerte derjenigen Detektoren, die von dem aus dem Patienten 4 austretenden Röntgenstrahlenbündel 3 getroffen werden, mittels der Messwerterfassungseinheit 14 erfasst und dem Rechner 18 zugeführt. Der Rechner 18 kann aus den Messwerten und der Information über den Querschnitt des Gefässes 21 die Kontrastmittelkonzentration bestimmen. Ferner ist es möglich, auf diese Weise die Kontrastmittelmenge zu ermitteln. Die vom Rechner 18 ermittelten Daten können z.B. auf einem Bildschirm sowohl graphisch als auch als Zahlenwerte wiedergegeben werden.

Zusätzlich zur Blende 2 kann auch zwischen dem Patienten 4 und dem Strahlenempfänger 5 eine entsprechende Blende angeordnet werden.

### Patentanspruch

Schichtaufnahmegerät zur Herstellung von Transversalschichtbildern eines Aufnahmeobjektes (4), mit einer Patientenliege (6), einer Strahlenmessanordnung (1, 5) mit einer Strahlenquelle (1), die ein das Aufnahmeobjekt (4) durchdringendes Strahlenbündel (3, 3a) erzeugt, dessen Querschnittsausdehnung senkrecht zur Schichtebene gleich der Schichtstärke ist und mit einem aus einer in der Schichtebene liegenden Reihe von Detektorelementen bestehenden Strahlenempfänger (5) mit nachgeschalteter Messwerterfassungseinheit (14), der die Strahlungsintensität hinter dem Objekt (4) ermittelt, mit einer Antriebsvorrichtung (9, 10) für die Messanordnung (1, 5) mit einem Drehgestell (9) zur Erzeugung von Drehbewegungen der Strahlenmessanordnung (1, 5) und mit einem Messwertumformer (15) für die Transformation der vom Strahlenempfänger (5) gelieferten Signale in ein Schichtbild, bei dem Mittel (8, 9) zum Verriegeln der Messanordnung (1, 5) gegen Drehung und zum Verriegeln der Patientenliege (6) gegen Längsverschiebung vorhanden sind, dadurch gekennzeichnet, dass zum Zweck der Untersuchung der Kontrastmittelkonzentration und ihres zeitlichen Verlaufes ein zusätzlicher Rechner (18) vorhanden ist, der aus den Messwerten der Messwerterfassungseinheit (14) und einer Information über den erfassten Querschnitt die Kontrastmittelkonzentration bestimmt, dass eine verstellbare Blende (2) vorhanden ist zum Eingrenzen des Strahlenbündels (3) in der Schichtebene und Auswählen eines Teiles der Detektoren, und dass eine Steuereinrichtung (20) für die Blende (2) vorgesehen ist, welche Steuereinrichtung (20) mit dem zusätzlichen Rechner (18) zusammenarbeitet, und dass alles derart ausgebildet ist, dass der Rechner (18) die Kontrastmittelkonzentration bestimmt bei verriegelter Messanordnung (1, 5), verriegelter Patientenliege (6) und eingestellter Blende (2).

### Claim

An X-ray device for the production of transverse tomographic images of an object to be photographed (4), comprising a patients' couch (6), a radiation-measuring arrangement (1, 5) with a radiation source (1) which produces a beam (3, 3a) whose cross-section at right angles to the layer plane corresponds to the layer thickness, and comprising a radiation receiver (5) consisting of a row of detector elements arranged in the layer plane and a subsequent measured value detection unit (14) to determine the radiation intensity behind the object (4), comprising a drive device (9, 10) for the measuring arrangement (1, 5), a pivot mounting unit (9) for producing rotary motions of the radiation-measuring arrangement (1, 5) and comprising a measured value converter (15) for the transformation of the signals supplied by the radiation receiver (5) into a tomographic image means (8, 9) being provided for locking the measuring arrangement (1, 5) against rotation and for locking the patients' couch (6) against longitudinal displacement, characterised in that for the purpose of examining the mean contrast concentration and its variation with time an additional computer (18) is arranged which determines the mean contrast concentration from the measured values of the measured value detection unit (14) and an item of information about the covered cross-section, that an adjustable diaphragm (2) is provided for locating the beam (3) in the layer plane and selecting part of the detectors, and that a control device (20) is provided for the diaphragm (2), which control device (20) cooperates with the additional computer (18), and that all is designed such that the computer (18) determines the mean contrast concentration when the measuring arrangement (1, 5) is locked, the patients' couch (6) is locked and the diaphragm (2) is adjusted.

### Revendication

Appareil de tomographie pour la réalisation de tomographies transversales d'un objet de prise de vues (4), comportant une couchette pour patient (6), un dispositif (1, 5) de mesure du rayonnement comportant une source de rayonnement (1), qui produit un faisceau de rayonnement (3, 3a) traversant l'objet de prise de vues (4) et dont l'étendue en coupe transversale perpendiculairement au plan de la couche est égale à l'épaisseur de la couche, et comportant un récepteur de rayonnement (5), constitué par une rangée d'éléments détecteurs, située dans le plan de la couche, et en aval duquel est branchée une unité (14) de détection de valeurs de mesure, qui détermine l'intensité du rayonnement derrière l'objet (4), et comportant un dispositif d'entraînement (9, 10) pour le dispositif de mesure (1, 5), muni d'un châssis tournant (9) servant à produire des mouvements de rotation du dispositif de mesure de rayonnement (1, 5) et un transformateur de valeurs de mesure (15) servant à transformer les signaux délivrés par le récepteur de rayonnement (5) en une tomographie, et dans lequel il est prévu des moyens (8, 9) servant à bloquer le dispositif de mesure (1, 5) contre toute rotation et à bloquer la couchette pour patient (6) contre tout déplacement longitudinal, caractérisé par le fait que pour examiner la concentration de l'agent de contraste et sa variation dans le temps, il est prévu un calculateur supplémentaire (18) qui détermine la concentration de l'agent de contraste à partir des valeurs de mesure de l'unité (14) de détection des valeurs de mesure et à partir d'une information concernant la section transversale détectée, qu'il est prévu un diaphragme réglable (2) servant à limiter le faisceau de rayonnement (3) dans le plan de la couche et à sélectionner une partie des détecteurs, et qu'il est prévu, pour le diaphragme (2), un dispositif de commande (20) qui opère en coopération avec le calculateur supplémentaire (18), et que l'ensemble est agencé de telle sorte que le calculateur (18) détermine la concentration de l'agent de contraste lorsque le dispositif de mesure (1, 5) est bloqué, que la couchette pour patient (6) est bloquée et que le diaphragme (2) est réglé.